# EUROPÄISCHE PATENTANMELDUNG

(11) **EP 3 834 778 A1**
(43) Veröffentlichungstag der Anmeldung: **16.06.2021**
(21) Anmeldenummer: 19216226.1
(22) Anmeldetag: 13.12.2019
(51) Int. Cl.: A61F 2/28, A61F 2/30, A61B 17/80, A61B 17/00

(54) **IMPLANTAT ZUR BEHANDLUNG VON KNOCHEN**

(71) Anmelder: Medartis Holding AG, 4057 Basel (CH)
(72) Erfinder: Mullis, Andreas, 4465 Hemmiken (CH)
(74) Vertreter: Hepp Wenger Ryffel AG

(57) **Zusammenfassung**

Die Erfindung betrifft ein Implantat (1) zur Behandlung von Knochen, insbesondere zur Abdeckung von Defekten oder Bohrlöchern oder zur Rekonstruktion von Knochendefekten oder Fehlbildungen. Dieses umfasst mindestens eine Rahmenstruktur (2) und mindestens einen Anpassungsbereich (3). Der Rand des Implantates (4) wird dabei teilweise, aber nicht durchgehend, durch die Rahmenstrukturen (2) gebildet, die sich ausserhalb des Anpassungsbereiches (3) befinden.

## Beschreibung

Die Erfindung betrifft ein Implantat zur Behandlung von Knochen mit den Merkmalen des Oberbegriffes des unabhängigen Anspruches.

Knochenfrakturen, insbesondere am menschlichen Schädel, können bekanntermassen mit Implantaten behandelt werden. Ziel der Behandlung und der Implantierung eines Implantates ist im Allgemeinen, die anatomisch korrekte Form und Lage der zu behandelnden Knochen und deren Umgebung wiederherzustellen und zu erhalten. Dadurch wird der Heilungsprozess gefördert und nach erfolgter Heilung die Funktionalität des betroffenen Körperteils erhalten.

Im Stand der Technik sind diverse Implantate bekannt, die an bestimmte Körperpartien angepasst sind.

EP 2 030 596 offenbart ein Implantat zur Behandlung von Frakturen der Augenhöhle. Diese umfassen einen flächigen Anpassungsbereich sowie eine Rahmenstruktur.

US 5,139,497 offenbart ein Orbitabodenimplantat mit einer Rahmenstruktur und einem Gitter.

US 2007/0238069 offenbart zuschneidbare und verformbare Meshes zur Behandlung von Gesichtsfrakturen.

Die bekannten Implantate weisen aber verschiedene Nachteile auf. So sind beispielsweise gewisse Implantate mit Rahmenstrukturen ausgestattet, die an bestimmte Knochenpartien angepasst sind. Dadurch ist die Implantierung des Implantates aber zuweilen schwierig, insbesondere da die exakte Anatomie von Menschen natürlich variiert. Gleichzeitig ist aber eine gewisse Anpassung an die Anatomie wünschenswert.

Es ist daher Aufgabe der vorliegenden Erfindung, die Nachteile des Bekannten zu vermeiden, insbesondere ein Implantat zur Verfügung zu stellen, das einerseits an die Anatomie der zu behandelnden Körperpartie angepasst ist aber gleichzeitig gewisse Anpassungen erlaubt.

Erfindungsgemäss werden diese und andere Aufgaben mit einem Implantat mit den Merkmalen des unabhängigen Anspruches gelöst.

Das erfindungsgemässe Implantat ist besonders geeignet zur Abdeckung von Defekten oder Bohrlöchern oder zur Rekonstruktion von Knochendefekten oder Fehlbildungen. Es umfasst mindestens eine Rahmenstruktur sowie einen flächigen Anpassungsbereich. Die Rahmenstruktur ist dabei ausserhalb des Anpassungsbereiches angeordnet. Sie bildet so teilweise den Rand des Implantates. Dabei begrenzt die Rahmenstruktur den äusseren Rand aber nicht durchgehend. Dadurch ist mindestens ein Bereich des äusseren Randes nicht durch die Rahmenstruktur begrenzt. Dadurch kann die Rahmenstruktur des Implantates an eine gewünschte Anatomie angepasst werden. Gleichzeitig erlaubt der nicht durch die Rahmenstruktur begrenzte Anpassungsbereich einen Zuschnitt oder eine Verformung und damit eine weitere Anpassung an die Anatomie.

Unter einer Rahmenstruktur soll ein Bereich des Implantats, insbesondere ein Randbereich, verstanden werden, der durch eine Dimensionierung und/oder durch Materialauswahl und/oder Formgebung so ausgeführt ist, dass eine Minimalkraft, die zu einer plastischen Verformung des Randbereichs, insbesondere bevorzugt des Randbereiches und des Anpassungsbereiches zusammen, erforderlich ist, höher ist als eine Minimalkraft, die zu einer plastischen Verformung des Anpassungsbereichs erforderlich ist. Diese Eigenschaft wird hier und im Folgenden als Biegbarkeit bezeichnet. Eine kleinere Biegbarkeit eines Teiles bedeutet daher, dass eine grössere Kraft aufgewendet werden muss, um es plastisch zu verbiegen. Eine grössere Biegbarkeit eines Teiles bedeutet, dass eine kleinere Kraft ausreicht, um es plastisch zu verformen. Dementsprechend kann die Rahmenstruktur im Vergleich zum Anpassungsbereich weniger biegbar sein. Zusätzlich oder alternativ kann eine Rahmenstruktur auch ein Bereich des Implantats sein, insbesondere ein Randbereich, der die Periodizität des Gitters unterbricht.
Das erfindungsgemässe Implantat ist insbesondere zur Behandlung der menschlichen Stirnhöhle geeignet und entsprechend geformt und dimensioniert. Selbstverständlich kann es bei geeigneter Grösse und Form aber auch für andere Körperpartien verwendet werden.

Besonders bevorzugt ist das Implantat spiegelsymmetrisch entlang einer Spiegelebene ausgeführt.

Bevorzugt umfasst das Implantat mindestens zwei Rahmenstrukturen. Diese sind beide jeweils ausserhalb des Anpassungsbereiches angeordnet und bilden so teilweise den Rand des Implantates. Die beiden Rahmenstrukturen begrenzen den äusseren Rand des Implantates nicht durchgehend. Dadurch sind mindestens zwei Bereiche des äusseren Randes nicht durch die Rahmenstruktur begrenzt. Dies erlaubt insbesondere die Anpassung an Knochen, die zwei ähnliche und/oder symmetrische Bereiche aufweisen, wobei der Zwischenbereich von Mensch zu Mensch variiert. Beispielsweise können die beiden Rahmenstrukturen an die Bereiche um die Augen angepasst sein und einen Anpassungsbereich zur Anpassung an das Nasenbein aufweisen.

Bevorzugt ist der Anpassungsbereich durchgehend ausgebildet und ist im Inneren frei von Rahmenstrukturen.

Bevorzugt ist die Rahmenstruktur so dimensioniert und angeordnet, dass mindestens die Hälfte des äusseren Randes nicht von der Rahmenstruktur begrenzt wird. Die Hälfte des äusseren Randes bedeutet vorliegend die Hälfte der Länge des äussersten Randes des Implantates.

Bevorzugt umfasst der Anpassungsbereich eine Gitterstruktur. Diese ist so dimensioniert, dass sie von Hand oder mit Handwerkzeugen verformt werden kann. Insbesondere kann der Anpassungsbereich so ausgebildet sein, dass seine Biegbarkeit entsprechend angepasst ist.

Bevorzugt umfasst das Implantat ein biokompatibles Material, insbesondere aus der Gruppe von Implantatstahl und/oder andere Metalle, Keramiken und Kunststoffe. Besonders bevorzugt umfasst das Implantat Titan oder eine Titanlegierung.

Bevorzugt weist der Anpassungsbereich Verbindungsstellen auf, die so dimensioniert sind, dass sie mittels Handwerkzeugen durchtrennt werden können. Dadurch wird einem Operateur ermöglicht, den Anpassungsbereich einfach zuzuschneiden und so an die Anatomie des Patienten anzupassen.

Der Anpassungsbereich umfasst bevorzugt eine Struktur aus Löchern, deren Umfangsbereiche über Verbindungsstellen miteinander verbunden sind. Insbesondere können die Verbindungsstellen als Stege ausgeführt sein, die die Umfangsbereiche der Löcher in regelmässigen Abständen entlang ihres Umfanges verbinden.

Bevorzugt weist das Implantat eine Seitenlänge in einem Bereich von 10-200 mm auf. Alternativ kann die Seitenlänge auch in einem Bereich von 30-70 mm, oder 25-50 mm, liegen. Insbesondere kann das Implantat eine annähernd quadratische Form mit abgerundeten Eckbereichen aufweisen. Besonders bevorzugt ist die Rahmenstruktur in zwei benachbarten, abgerundeten Eckbereichen angeordnet.

Bevorzugt ist mindestens eine Rahmenstruktur so dimensioniert und positioniert, dass sie auf der Margo Supraorbitalis angebracht, insbesondere angeschraubt, werden kann.

Bevorzugt weist die Rahmenstruktur eine Bogenform auf. Insbesondere kann die Bogenform einen Krümmungsradius von 10-200 mm, besonders bevorzugt von 10-80 mm, aufweisen. Der Krümmungsradius kann in den Bereichen der Rahmenstruktur variieren. Beispielsweise kann der Krümmungsradius entlang einer Randstruktur kontinuierlich zunehmen, so dass die Rahmenstruktur die Form einer Klothoide bildet.

Besonders bevorzugt weist das Implantat einen Zwischenbereich auf. Dieser ist zwischen den beiden Rahmenstrukturen, die an die Margo Orbitalis angepasst sind, angeordnet und weist eine grössere Biegbarkeit als die beiden Rahmenstrukturen auf.

Bevorzugt weist das Implantat mindestens eine Befestigungslasche auf. Alternativ kann das Implantat auch mindestens zwei Befestigungslaschen aufweisen. Insbesondere kann die Befestigungslasche am Rand des Implantates oder an einer Rahmenstruktur angeordnet sein. Bevorzugt weist die Befestigungslasche auch Schraublöcher auf. Dadurch kann das Implantat auf besonders vorteilhafte Weise fixiert, beispielsweise an einen Knochen angeschraubt, werden. Bevorzugt erstreckt sich die Befestigungslasche in derselben Ebene wie das Implantat vom Rand des Implantates weg, insbesondere in einem Azimutwinkel zwischen 70° und 95° bezogen auf den Rand. Insbesondere wenn der Rand des Implantates an der Stelle, wo eine Befestigungslasche angeordnet ist, nicht gerade ist, soll die Azimutwinkelangabe als der Winkel zwischen der Befestigungslasche und einer Tangente zum Rand des Implantates in dem Punkt, wo die Befestigungslasche angeordnet ist, verstanden werden. Besonders bevorzugt erstrecken sich zwei Befestigungslaschen derart vom Implantat weg, dass zwischen den Befestigungslaschen ein spitzer Winkel entsteht, der sich vom Implantat weg öffnet.

Zusätzlich oder alternativ können eine oder mehrere Befestigungslaschen auch so angeordnet sein, dass sie nicht in derselben Ebene liegen wie das Implantat, wie der Rand des Implantates, oder wie der Anpassungsbereich des Implantates und daher in einem Elevationswinkel zu der erwähnten Ebene liegen. Die Befestigungslasche kann insbesondere von der Ebene, in der zumindest der Rand des Implantates und/oder eine Rahmenstruktur liegt, abgewinkelt sein.

Dabei versteht es sich, dass auch Implantate denkbar sind, die nicht flach ausgebildet sind, sondern eine Freiform aufweisen. Insbesondere kann das Implantat lokale Biegungen zwischen flächigen Elementen aufweisen, und/oder eine Form einer Rotations- oder Translationfläche aufweisen. In diesem Fall bezieht sich die vorstehend genannte Ebene auf eine Tangentenfläche an die Oberfläche des Implantates am Ort der Befestigung der Lasche. Insbesondere bei Laschen, die nicht in derselben Ebene liegen wie die Tangentenfläche, kann ein Elevationswinkel daher zwischen der Lasche und ihrer Projektion auf der Tangentenfläche gemessen werden. Ein Azimutwinkel kann in der Tangentenebene zwischen der Lasche und einer Tangente an den Rand des Implantates gemessen werden.

Insbesondere kann die mindestens eine Befestigungslasche einstückig mit der Rahmenstruktur verbunden sein. Das Implantat kann auch mindestens zwei Befestigungslaschen aufweisen, die einstückig mit der Rahmenstruktur verbunden sind. Falls das Implantat auch mindestens zwei Rahmenstrukturen aufweist, sind die mindestens zwei Befestigungslaschen bevorzugt mit jeweils einer Rahmenstruktur einstückig verbunden. Es ist aber auch möglich, dass mehr als eine Befestigungslasche mit der gleichen Rahmenstruktur einstückig verbunden ist.

Alternativ ist aber auch möglich, eine oder mehrere Befestigungslaschen am Rand des Implantates anzuordnen, ohne sie mit der Rahmenstruktur zu verbinden. Insbesondere können eine oder zwei Befestigungslaschen zwischen zwei Rahmenstrukturen angeordnet werden. Dies ist besonders vorteilhaft, wenn mindestens eine Rahmenstruktur so dimensioniert und positioniert ist, dass sie auf der Margo Supraorbitalis angebracht, insbesondere mit Schrauben befestigt, werden kann.

Bevorzugt ist die mindestens eine Befestigungslasche zur Befestigung am Nasenbein angepasst. Die Anpassung kann dabei insbesondere durch die Dimension, Form, und Positionierung am Implantat erfolgen. Wenn das Implantat mindestens zwei Befestigungslaschen aufweist, sind bevorzugt auch mindestens zwei Befestigungslaschen auf gleiche Weise zur Befestigung am Nasenbein angepasst.

Bevorzugt ist der Anpassungsbereich einstückig ausgebildet.

Bevorzugt ist das ganze Implantat einstückig ausgebildet.

Bevorzugt ist das Implantat zur Abdeckung von Defekten oder Bohrlöchern oder zur Rekonstruktion von Knochendefekten oder Fehlbildungen an der Stirnhöhle angepasst.

Bevorzugt ist der Anpassungsbereich plastisch deformierbar. Insbesondere kann der Anpassungsbereich so dimensioniert oder in der Materialauswahl angepasst sein, dass eine plastische Deformation möglich ist.

Die Erfindung wird nachfolgend anhand der Figuren und Ausführungsbeispiele näher erläutert. Es zeigen:
- Fig. 1:: eine Ausführungsform eines erfindungsgemässen Implantates,
- Fig. 2:: eine alternative Ausführungsform eines erfindungsgemässen Implantates,
- Fig. 3:: eine weitere alternative Ausführungsform eines erfindungsgemässen Implantates,
- Fig. 4:: eine vergrösserte Darstellung eines Anpassungsbereiches,
- Fig. 5:: eine alternative Ausführungsform eines erfindungsgemässen Implantates.

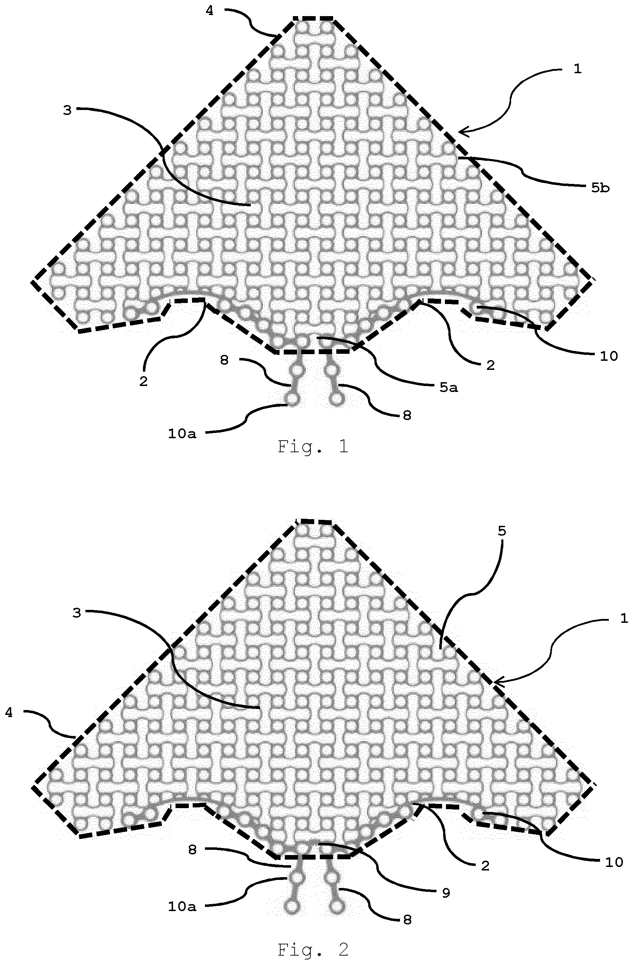

Fig. 1 zeigt eine Ausführungsform eines erfindungsgemässen Implantates 1. Dieses ist in Form und Grösse angepasst, um im Bereich der menschlichen Stirnhöhle implantiert zu werden. Das Implantat umfasst zwei Rahmenstrukturen 2 und einen Anpassungsbereich 3. Der äussere Rand des Implantates ist durch eine gestrichelte Linie 4 symbolisiert und soll hier und im Allgemeinen als die äusserste Grenze des gesamten Implantates vor einem allfälligen Zuschnitt verstanden werden. Die beiden Rahmenstrukturen 2 bilden teilweise den äusseren Rand 4 des Implantates. Insbesondere weisen die Rahmenstrukturen 2 hier eine Bogenform auf und sind in ihrer Form und Dimension der menschlichen Margo Supraorbitalis angepasst. Weiter umfassen sie Schraublöcher 10, die zur Aufnahme von Schrauben geeignet sind, so dass das Implantat 1 an einen Knochen geschraubt werden kann. Das Implantat umfasst weiter zwei Befestigungslaschen 8. Diese sind mit jeweils einer der Rahmenstrukturen 2 einstückig verbunden. Vorliegend sind die Befestigungslaschen 8 an den einander zugewandten Enden der Rahmenstrukturen 2 zwischen den beiden Rahmenstrukturen verbunden und erstrecken sich in derselben Ebene wie das Implantat in einem Winkel von ungefähr 85° vom Implantat weg. Dadurch entsprechen die Befestigungslaschen 8 der Position des Nasenbeins, wenn die Rahmenstrukturen auf der Margo Supraorbitalis angebracht werden. Weiter umfassen die Befestigungslaschen Schraublöcher 10a, die für eine Verschraubung der Befestigungslaschen am Nasenbein geeignet sind. Zwischen den beiden Rahmenstrukturen 2 und Befestigungslaschen befindet sich ein Zwischenbereich 5a, der keine Rahmenstruktur aufweist. Dieser entspricht in seiner Ausführung dem restlichen Randbereich 5b des Implantates und ist insbesondere so ausgeführt, dass er eine grössere Biegbarkeit aufweist als die Rahmenstruktur. Dadurch ist die benötigte Kraft, um eine plastische Deformation des Zwischenbereiches 5a zu bewirken, relativ klein und kann von Hand oder durch Handwerkzeuge aufgebracht werden. Vorliegend umfasst das Implantat nur Rahmenstrukturen, die an den Bereich der Margo Supraorbitalis angepasst sind und über Befestigungslaschen am Nasenbein angebracht werden können. Der Rest 5b des äusseren Randes 4 ist nicht von Rahmenstrukturen begrenzt und kann daher durch den Operateur zugeschnitten werden. Dadurch kann beispielsweise das Implantat auf einen kleineren Bereich der Anatomie eines Patienten angepasst werden. Zusätzlich ist der Anpassungsbereich 3 plastisch verformbar, so dass weitere Anpassungsmöglichkeiten bestehen.

Fig. 2 zeigt eine alternative Ausführungsform eines erfindungsgemässen Implantates 1. Die hier gezeigt Ausführungsform umfasst nur eine Rahmenstruktur 2. Diese begrenzt den äusseren Rand 4 teilweise, so dass ein Teil 5 des äusseren Randes ohne Rahmenstruktur ausgebildet ist. Der flächige Anpassungsbereich 3 ist im Wesentlichen gleich ausgebildet wie derjenige in Fig. 1. Er ist daher plastisch verformbar und weist eine Biegbarkeit auf, die eine plastische Verformung von Hand oder mit Handwerkzeugen erlaubt. Die Rahmenstruktur 2 ist hier so angepasst, dass sie in Form und Dimensionierung der Margo Supraorbitalis entspricht. Das Implantat weist zwei Befestigungslaschen 8 auf, die beide einstückig mit der Rahmenstruktur 2 verbunden sind. Die Befestigungslaschen sind so dimensioniert und lokalisiert, dass sie auf dem Nasenbein angebracht werden können. Dazu dienen insbesondere die Schraublöcher 10a, die zur Verschraubung am Nasenbein genutzt werden können. Die Rahmenstruktur umfasst ebenfalls Schraublöcher 10, die demselben Zweck dienen. Die Rahmenstruktur 2 ist hier zwischen den beiden Befestigungslaschen 8 durchgehend ausgebildet. Daher ist der Zwischenbereich 9 zwischen den Befestigungslaschen Teil der Rahmenstruktur 2. Dies ist besonders vorteilhaft, wenn die Behandlung ein Stützung und Stabilisierung in diesem Bereich erfordert und/oder die Form und Dimensionierung des Implantates so genau auf die Anatomie angepasst ist, dass sich eine weitere Anpassung erübrigt. Unabhängig davon lässt sich aber natürlich der Anpassungsbereich 3 plastisch verformen und/oder zuschneiden, um einer besonderen Anatomie Rechnung zu tragen.

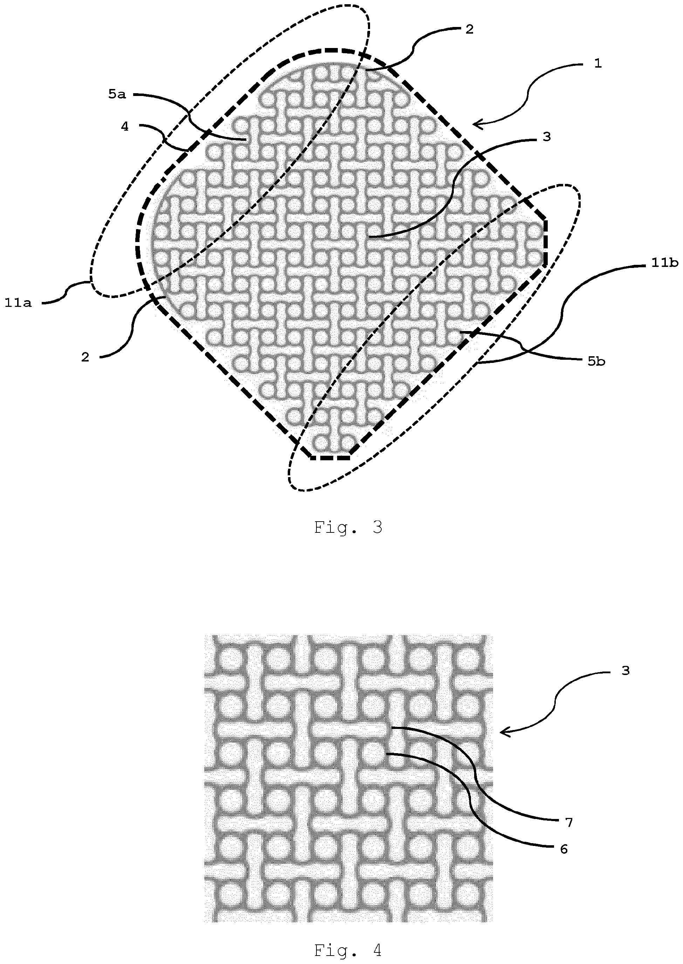

Fig. 3 zeigt eine alternative Ausführungsform eines erfindungsgemässen Implantates 1. Dieses weist zwei Rahmenstrukturen 2 auf, die den äusseren Rand 4 des Implantates teilweise bilden. Dadurch umfasst das Implantat zwei weitere Bereiche 5a, 5b des äusseren Randes 4, die nicht durch Rahmenstrukturen begrenzt sind. Der flächige Anpassungsbereich 3 ist auch hier so ausgeführt, dass er plastisch verformbar und zuschneidbar ist. Insbesondere ist er so dimensioniert und ausgeführt, beispielsweise durch Materialauswahl und/oder Form, dass die plastische Verformung und/oder der Zuschnitt von Hand oder mit Handwerkzeugen erfolgen kann. Geeignete Handwerkzeuge umfassen insbesondere handelsübliche Zangen und Schneidinstrumente. Das Implantat weist eine annähernd quadratische Form mit abgerundeten Eckbereichen mit einem Radius auf, wo auch die Rahmenstrukturen angeordnet sind. Dadurch umfasst das vorliegende Implantat einen Bereich 11a, der insbesondere durch die Rahmenstruktur keine scharfen Stellen oder Ausfransungen aufweist. Ein zweiter Bereich 11b, der im vorliegenden Beispiel dem Bereich 11a gegenüberliegt, ist wegen der fehlenden Rahmenstrukturen besonders geeignet, um durch einen Operateur auf eine kleinere Grösse zugeschnitten zu werden. Bevorzugt erfolgt der Zuschnitt auf der den Rahmenstrukturen abgewandten Seite. Es ist selbstverständlich aber auch möglich, den Anpassungsbereich auf eine beliebige Form zuzuschneiden, insbesondere auch zwischen den beiden Rahmenstrukturen. Dadurch entsteht ein kleineres Implantat mit nur einer Rahmenstruktur. Durch die Flexibilität des Zuschneidens ist dieses Implantat besonders geeignet für den Einsatz, wo eine genaue Anpassung des Implantats vor der Operation nicht oder nur schwierig möglich ist und deswegen Flexibilität in der Anwendung wichtig ist. Beispielsweise ist dies bei der Behandlung von Knochen der Fall, die typischerweise eine grosse Variation in Grösse und Form zwischen einzelnen Menschen aufweisen.

Es wäre alternativ natürlich denkbar, das Implantat auch innerhalb der Rahmenstruktur zuzuschneiden. Die Rahmenstruktur muss daher nicht so ausgeführt sein, dass sie nicht zugeschnitten werden kann. Im Allgemeinen ist es aber vorteilhaft, die Rahmenstruktur so anzuordnen, dass sie bei den häufigsten Anwendungsfällen nicht zugeschnitten werden muss. So bildet die Rahmenstruktur einen Bereich ohne scharfe Kanten.

Fig. 4 zeigt eine Detaildarstellung des flächigen Anpassungsbereiches 3 und der Geometrie des Gitters. Das vorliegende Gitter umfasst Löcher 6 und als Stege ausgeführte Verbindungsstellen 7. Die Verbindungsstellen verbinden Umfangsbereiche der Löcher 6. In der hier dargestellten Ausführungsform ist jeder Umfangsbereich eines Loches 6 mit vier Verbindungsstellen 7 verbunden. Diese sind entlang des Umfanges der Löcher gleichmässig, das heisst ungefähr in 90°-Abständen, verteilt. Die Verbindungsstellen sind weiter so dimensioniert, dass sie mit Handwerkzeugen durchtrennt werden können. Hier weisen die Löcher einen Aussendurchmesser von 3.1 mm auf. Es wäre aber auch denkbar, Löcher mit einem anderen Aussendurchmesser zu bilden, insbesondere einen Aussendurchmesser im Bereich von 2 bis 5 mm, bevorzugt in einem Bereich von 3.0 bis 3.22 mm. Die drahtartigen Elemente, die die Stege sowie die Umfangsbereiche der Löcher bilden, weisen eine Breite von 0.6 mm auf, könnten alternativ aber auch eine andere Breite im Bereich 0.1 bis 3.0 mm, bevorzugt 0.5 bis 0.7 mm, aufweisen. Der Gitterbereich ist vorliegend ungefähr 0.5 mm dick, könnte aber auch in einer andere Dicke im Bereich von 0.1 bis 2.0 mm, bevorzugt 0.3 bis 0.6 mm, ausgeführt werden. Besonders vorteilhaft ist die Ausführung des Gitters aus einem Metall oder einem (resorbierbaren) Kunststoff. Dementsprechend besteht die hier dargestellte Variante aus Titan oder einer TitanLegierung. Durch diese Ausführung lässt sich der flächige Anpassungsbereich auf eine gewünschte Grösse zuschneiden. Selbstverständlich können aber andere Dimensionen und/oder Materialien zur Anwendung kommen. Die hier dargestellte Geometrie des Gitters, das den flächigen Anpassungsbereich 3 bildet, ist aufgrund der hier beschriebenen Eigenschaften besonders vorteilhaft für die Anwendung in einem Implantat. Es ist aber selbstverständlich auch möglich, irgendeine andere bekannte Gitterstruktur zu verwenden.

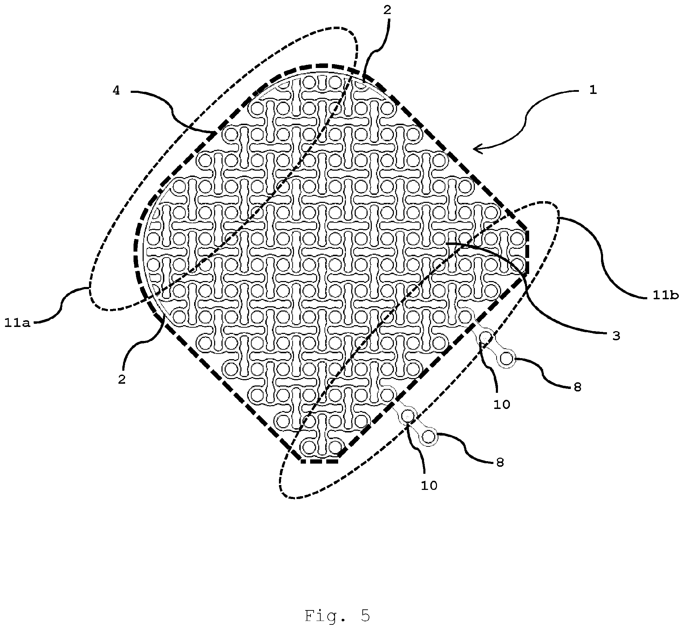

Fig. 5 zeigt eine alternative Ausführungsform eines erfindungsgemässen Implantates. Dieses entspricht im Wesentlichen der in Fig. 3 gezeigten Ausführungsform, umfasst aber weiter zwei Befestigungslaschen 8. Diese sind ähnlich den Befestigungslaschen, wie sie beispielsweise in der in Fig. 1 gezeigten Ausführungsform eingesetzt werden. In der hier gezeigten Ausführungsform sind die Befestigungslaschen 8 aber nicht mit einer Rahmenstruktur verbunden. Stattdessen sind beide Befestigungslaschen 8 direkt mit dem Randbereich 5b des Implantates 1 verbunden und befinden sich daher in einem Bereich mit einer grösseren Biegbarkeit des Implantates. Dadurch kann das Implantat 1 besonders vorteilhaft bei komplizierten Anatomien verwendet werden, weil die Befestigungslaschen 8 mit maximaler Flexibilität fixiert werden können. Zu diesem Zweck verfügen die Befestigungslaschen 8 ebenfalls über Schraublöcher 10. Es wäre natürlich möglich, die in den Figuren 1 und 5 gezeigte Anordnung der Befestigungslaschen 8 zu kombinieren. So könnte auch die hier gezeigte Variante zusätzlich oder alternativ weitere Befestigungslaschen 8 im Bereich der Rahmenstrukturen umfassen. Ebenso könnte die in Fig. 1 gezeigte Ausführungsform mit weiteren Befestigungslaschen versehen werden, die nicht mit der Rahmenstruktur verbunden sind.

## Patentansprüche

1. Implantat (1) zur Behandlung von Knochen, insbesondere zur Abdeckung von Defekten oder Bohrlöchern oder zur Rekonstruktion von Knochendefekten oder Fehlbildungen, umfassend
- mindestens eine Rahmenstruktur (2)
- mindestens einen insbesondere flächigen Anpassungsbereich (3)
wobei die mindestens eine Rahmenstruktur (2) ausserhalb des Anpassungsbereiches (3) angeordnet ist und teilweise den Rand des Implantats (4) bildet,
**dadurch gekennzeichnet, dass**
die mindestens eine Rahmenstruktur (2) den äusseren Rand (4) nicht durchgehend begrenzt, so dass mindestens ein Bereich des äusseren Randes (5) nicht durch die Rahmenstruktur begrenzt wird.

2. Implantat (1) gemäss Anspruch 1, **dadurch gekennzeichnet, dass** das Implantat mindestens zwei Rahmenstrukturen (2) umfasst, wobei beide Rahmenstrukturen (2) jeweils ausserhalb des Anpassungsbereiches (3) angeordnet sind und teilweise den Rand des Implantats (4) bilden, und die mindestens zwei Rahmenstrukturen (2) den äusseren Rand (4) nicht durchgehend begrenzen, so dass mindestens zwei Bereiche des äusseren Randes (5a, 5b) nicht durch die Rahmenstruktur begrenzt werden.

3. Implantat (1) gemäss einem der Ansprüche 1 oder 2, **dadurch gekennzeichnet, dass** der Anpassungsbereich (3) durchgehend ausgebildet ist und im Inneren frei von Rahmenstrukturen (2) ist.

4. Implantat (1) gemäss einem der Ansprüche 1 bis 3, **dadurch gekennzeichnet, dass** die Rahmenstrukturen so dimensioniert und angeordnet sind, dass mindestens die Hälfte des äusseren Randes (4) durchgehend nicht von den Rahmenstrukturen (2) begrenzt wird.

5. Implantat (1) gemäss einem der Ansprüche 1 bis 4, **dadurch gekennzeichnet, dass** der Anpassungsbereich (3) eine Gitterstruktur (6) umfasst, die so dimensioniert ist, insbesondere eine derartige Biegbarkeit aufweist, dass sie von Hand oder mit Handwerkzeugen verformt werden kann.

6. Implantat (1) gemäss einem der einem der Ansprüche 1 bis 5, **dadurch gekennzeichnet, dass** das Implantat (1) ein biokompatibles Material umfasst, bevorzugt ein biokompatibles Material aus der Gruppe von Implantatstahl, Metallen, Keramiken, Kunststoffe, Verbundwerkstoffe, besonders bevorzugt Titan oder eine Titanlegierung.

7. Implantat (1) gemäss einem der Ansprüche 1 bis 6, **dadurch gekennzeichnet, dass** der Anpassungsbereich Verbindungsstellen (7) aufweist, die so dimensioniert sind, dass sie mittels Handwerkzeugen durchtrennt werden können.

8. Implantat (1) gemäss einem der Ansprüche 1 bis 7, **dadurch gekennzeichnet, dass** das Implantat mindestens eine Seitenlänge in einem Bereich von 10-200 mm, bevorzugt 30-70 mm, besonders bevorzugt 25-50 mm, aufweist.

9. Implantat (1) gemäss einem der Ansprüche 1 bis 8, **dadurch gekennzeichnet, dass** mindestens eine Rahmenstrukturen so dimensioniert und positioniert ist, dass sie auf der Margo Supraorbitalis angebracht, bevorzugt angeschraubt, werden kann.

10. Implantat (1) gemäss Anspruch 9, **dadurch gekennzeichnet, dass** die mindestens eine Rahmenstruktur (2) eine Bogenform, insbesondere eine Bogenform mit einem Krümmungsradius von 1 cm bis 20 cm, besonders bevorzugt mit einem Krümmungsradius von 1 cm bis 8 cm, aufweisen.

11. Implantat (1) gemäss Anspruch 9 oder 10, **dadurch gekennzeichnet, dass** das Implantat zwischen den beiden Rahmenstrukturen, die an die Margo Supraorbitalis angepasst sind, einen Zwischenbereich (5a)aufweist, dessen Biegbarkeit grösser ist als die der Rahmenstrukturen.

12. Implantat (1) gemäss einem der Ansprüche 1 bis 11, **dadurch gekennzeichnet, dass** das Implantat (1) mindestens eine Befestigungslasche (8), bevorzugt mindestens zwei Befestigungslaschen (8), umfasst.

13. Implantat (1) gemäss Anspruch 12, **dadurch gekennzeichnet, dass** die mindestens eine Befestigungslasche (8), bevorzugt die mindestens zwei Befestigungslaschen (8), einstückig mit einer Rahmenstruktur (2), bevorzugt mit je einer der mindestens zwei Rahmenstrukturen (2), verbunden ist.

14. Implantat (1) gemäss einem der Ansprüche 12 oder 13, **dadurch gekennzeichnet, dass** die mindestens eine Befestigungslasche (8), bevorzugt die mindestens zwei Befestigungslaschen (8), zur Befestigung am Nasenbein angepasst ist, insbesondere durch die Dimension, Form und Positionierung am Implantat (1) der mindestens einen Befestigungslasche (8), bevorzugt der mindestens zwei Befestigungslaschen (8).

15. Implantat (1) gemäss einem der Ansprüche 1 bis 14, **dadurch gekennzeichnet, dass** das Implantat zur Abdeckung von Defekten oder Bohrlöchern oder zur Rekonstruktion von Knochendefekten oder Fehlbildungen an der Stirnhöhle angepasst ist.

16. Implantat (1) gemäss einem der Ansprüche 1 bis 14, **dadurch gekennzeichnet, dass** der Anpassungsbereich (3) einstückig ausgebildet ist.

17. Implantat (1) gemäss einem der Ansprüche 1 bis 16, **dadurch gekennzeichnet dass** das ganze Implantat (1) einstückig ausgebildet ist.

18. Implantat (1) gemäss einem der Ansprüche 1 bis 17, **dadurch gekennzeichnet, dass** der Anpassungsbereich (3) plastisch deformierbar ist.
